# EUROPEAN PATENT SPECIFICATION

(11) **EP 3 967 209 B1**
(45) Date of publication and mention of the grant of the patent: **18.02.2026**
(21) Application number: 21196567.8
(22) Date of filing: 14.09.2021
(51) Int. Cl.: A61B 1/00, A61B 1/04, G02B 23/24

(54) **ENDOSCOPE FOR PERFORMING A RETROFLEXION MANOEUVRE AND MANUFACTURING METHOD THEREOF**
ENDOSKOP ZUR DURCHFÜHRUNG EINES RETROFLEXIONSMANÖVERS UND DESSEN HERSTELLUNGSVERFAHREN
ENDOSCOPE PERMETTANT D'EFFECTUER UNE MAN UVRE DE RÉTROFLEXION ET METHODE DE SA FABRRICATION

(30) Priority: 15.09.2020 DK PA202070592
(43) Date of publication of application: 16.03.2022
(73) Proprietor: Ambu A/S, 2750 Ballerup (DK)
(72) Inventor: RASK, Jesper Domino, 2300 Copenhagen S (DK)
(74) Representative: COPA Copenhagen Patents

(56) References cited:
- JP-A- 2011 056 014
- US-A- 4 872 446
- US-A1- 2001 056 224
- US-A1- 2008 004 492
- US-A1- 2011 004 057
- US-A1- 2011 245 612

## Description

### TECHNICAL FIELD

The present disclosure relates to an endoscope for performing a retroflexion manoeuvre and a method of making such an endoscope.

### BACKGROUND

Insertion endoscopes are well-known devices in the medical field for visually examining the interior of a hollow organ or cavity of a body, such as during urology procedures or gastrointestinal procedures, by means of inserting an insertion portion of the endoscope. The insertion portion of the endoscope comprises an elongated insertion tube, a distal tip part, and a bending section connecting the insertion tube with the distal tip part. The endoscope typically has a handle connected to the insertion tube and positioned at the proximal end of the endoscope as seen from the operator. The endoscope further has a vision device, such as a built-in camera or fibre optics. The vision device is typically incorporated in the distal tip part at the distal end of the endoscope. This definition of proximal as being closest to an operator and distal as being furthest from an operator is used throughout this disclosure. Illumination of the area in front of the distal tip part of the endoscope is normally required, in particular the field of vision of the vision device. One known way of achieving such illumination is to incorporate one or more Light Emitting Diodes (LEDs) in the distal tip part of the endoscope, e.g. as mentioned in WO2014/106511 disclosing a disposable endoscope. Alternatively, illumination may be provided by light guides and/or fibre optics guiding light from a light source outside the endoscope and to the distal tip part.

The bending section is provided in order to manoeuvre the endoscope inside the body cavity. The bending section has increased flexibility, e.g. achieved by a number of articulated segments of which the distal tip part forms the distalmost segment. Bending or straightening of the bending section in the insertion part of the endoscope is typically done by tensioning or slacking, respectively, steering wires running from the distal tip part through the remainder of articulated segments and along the inside of the elongated insertion tube to a control mechanism, such as a control lever, of the handle.

Data and/or power cables for the vision device (when being a camera) and other electronics, such as LED lighting accommodated in the distal tip part, also run along the inside of the elongated insertion tube and the bending section. Optionally, fibre optics/light guides for the illumination means, also run along the inside of the elongated insertion tube and the bending section from the handle to the distal tip part. Furthermore, a working channel may run along the inside of the insertion tube and the bending section from the handle to the tip part, e.g. allowing liquid to be removed from the body cavity or allowing the insertion of medical tools or surgical instruments into the body cavity.

In some procedures, a so-called retroflexion manoeuvre is performed in which the bending section bends backwards to allow the camera to inspect the course of the insertion tube and/or the anatomy adjacent to the insertion tube. One example is a urologic procedure, wherein the endoscope is inserted through the urethra and retroflexion is performed to inspect the area surrounding the urethra. Another example is a gastrointestinal procedure where it may also be necessary to perform inspection backwards by a retroflexion manoeuvre. During the retroflexion manoeuvre, the images produced by the vision device are often either over- or underexposed. When the images are overexposed, details are lost in the shadows and the darkest areas of the image. On the other hand, when the images are underexposed, details are lost in the highlights and the brightest parts of the image. Both cases can lead to poor image quality of portions of the image indicative of the anatomy of interest which makes it difficult for the operator to perform the inspection.

US 2001/056224 A1 discloses an endoscope comprising an end section having an increased bendability compared to a shaft of the endoscope. The end section 32 can be additionally bent by more than 180 degrees. Furthermore, document US 2008/004492 A1 discloses an endoscope of the prior art.

### SUMMARY

In light of the above, it may be seen as an object of the present disclosure to provide an endoscope with improved image quality during a retroflexion manoeuvre. Another object of the present disclosure is to provide a method of providing an endoscope with improved image quality during a retroflexion manoeuvre.

One or more of these objects may be met by aspects of the present disclosure as described in the following. The present application is defined in independent claims 1 and 11.

The present invention relates to an endoscope capable of performing a retroflexion manoeuvre, comprising:
- a handle having a control mechanism,
- a distal tip part having a vision device, such as a camera, with a field of view, and an illumination device, such as one or more light emitting diodes (LEDs), for providing illumination for the vision device,
- a bending section having a first state, a retroflexion state, and a distal end connected to the distal tip part,
- an insertion tube extending from the handle to a proximal end of the bending section and including an exterior surface facing the surroundings of the endoscope, and
- at least one steering wire connecting the control mechanism with the bending section so that manipulation of the control mechanism causes the endoscope to perform a retroflexion manoeuvre by bending the bending section from the first state to the retroflexion state,
wherein the exterior surface of the insertion tube includes a coloured area of a colour configured to reduce over- or underexposure of the vision device when the endoscope is in the retroflexion state.

By marking at least a portion of the area of the exterior surface of the insertion tube that is visible and illuminated during retroflexion with an optimised colour, the coloured area will appear neither too dark nor too bright in images produced by the vision device, thus allowing the vision device to optimise the exposure level and thereby provide the operator with an improved image quality at least of the portion of the image not covered by the coloured area, i.e. the portion of the image indicative of the tissue or anatomy.

Additionally or alternatively, the first state may be a resting state of the endoscope in which the bending section is preferably substantially straight.

Additionally or alternatively, the coloured area may be visible within the field of view of the camera in the retroflexion state of the bending section.

Additionally or alternatively, the field of view of the vision device may include a view cone with a view cone angle (α_{col}) of at least 10°, 20°, 30°, 40°, or 50° encompassing the coloured area within the field of view when the bending section of the endoscope is in the retroflexion state. The view cone is a subset of the field of view so the maximum view cone angle equals the field of view angle.

This may provide the advantage that when the bending section of the endoscope is in the retroflexion state, the majority of the insertion tube within the field of view of the vision device, in particular the part of the insertion tube closest to the vision device, is occupied by the coloured area instead of the non-coloured remainder of the insertion tube. This may have the advantage of positioning the coloured area where the improvement of the exposure level is greatest.

Additionally or alternatively, the colour of the coloured area may be different from a colour of a majority of the insertion tube.

By only marking the coloured area of the first section that is visible during retroflexion with an optimised colour, an improved image quality is provided while the colour of the majority of the insertion tube can be left the designers preference.

According to the present invention, the colour of the coloured area is a grey colour with L* between 15 to 85, preferably L* between 30 to 70, more preferably L* between 40 to 60, as measured by a CIE L*a*b* colour code system. More preferably, the colour may be a grey colour with a* between negative 10 to positive 10 and b* between negative 10 to positive 10.

Such a colour may have the advantage of improving the image quality when the bending section of the endoscope is in the retroflexion state by reducing over- or underexposure of the vision device.

Additionally or alternatively, a first section of the exterior surface of the insertion tube may be visible within the field of view of the vision device when the bending section of the endoscope is in the retroflexion state. The coloured area may form part of the first section and preferably covers at least 20%, 40%, 60%, 80%, 90%, or 100% of the area of the first section. Additionally or alternatively, the coloured area may cover up to 100%, 90%, 80%, 70%, 60%, or 40% of the area of the first section. In particular, the coloured area may cover an area in the range of 20% to 80% of the area of the first section, preferably 40% to 80%, more preferably 60% to 80%.

By providing the coloured area on the visible first section, the exposure level of the vision device may be further improved. The exposure level is further improved by increasing the coverage of the coloured area on the first section.

Additionally or alternatively, the first section may have a proximal portion and a distal portion. The coloured area may cover the distal portion of the first section. The distal portion may have a longitudinal extent of at most 90%, 80%, 70%, 60%, 50%, 40%, 30%, or 20% relative to a longitudinal extent of the exterior surface of the insertion tube. Additionally or alternatively, the coloured area may not cover the proximal portion of the first section.

By providing the coloured area on a distal portion of the insertion tube, the image quality can be improved while minimising the extent of the coloured area.

Additionally or alternatively, the exterior surface of the insertion tube may have a proximal portion and a distal portion. The coloured area may cover a distal portion of the insertion tube having an extent of at most 90%, 80%, 70%, 60%, 50%, 40%, 30%, or 20% relative to a longitudinal extent of the exterior surface. Additionally or alternatively, the coloured area may not cover the proximal portion of the exterior surface.

Additionally or alternatively, a bending angle of the bending section between the first state and the retroflexion state may be at least 180 degrees, preferably at least 210 degrees.

Additionally or alternatively, the insertion tube may comprise a plurality of depth marks, preferably spaced, optionally equidistantly, along the extent of the insertion tube. The depth marks may be of the same colour as the coloured area.

Such depth marks may allow the operator to easily infer how far the insertion tube has been inserted into a patient.

Additionally or alternatively, the endoscope may be a cystoscope, a ureteroscope or an endoscope used during urology procedures. Alternatively, the endoscope may be configured for use in gastrointestinal procedures, such as a duodenoscope, a gastroscope, a colonoscope.

Additionally or alternatively, the endoscope may be a disposable endoscope which may not be intended to be cleaned and/or sterilised for reuse.

A second aspect of this disclosure relates to an endoscope vision system comprising an endoscope according to the first aspect of this disclosure and a monitor, and wherein the endoscope is connectable to the monitor via one or more cables or via a wireless connection, e.g. a standard radiofrequency wireless connection such as Bluetooth, Wi-Fi, etc. The monitor includes a display unit configured for displaying images captured by the vision device of the endoscope.

The present invention further relates to a method for marking an area of an endoscope part for an endoscope capable of performing a retroflexion manoeuvre, the endoscope comprising:
- a handle having a control mechanism;
- a distal tip part having a vision device, such as a camera, with a field of view, and an illumination device, such as one or more light emitting diodes (LEDs), for providing illumination for the vision device;
- a bending section having a first state, a retroflexion state, and a distal end connected to the distal tip part;
- an insertion tube extending from the handle to a proximal end of the bending section and including an exterior surface facing the surroundings of the endoscope; and
- at least one steering wire connecting the control mechanism with the bending section so that manipulation of the control mechanism causes the endoscope to perform a retroflexion manoeuvre by bending the bending section from the first state to the retroflexion state;
wherein the method comprises the steps of:
- providing the insertion tube configured for insertion into a patient and including the exterior surface facing the surroundings, and
- locating an area of the exterior surface of the insertion tube being visible within a field of view of the vision device of the distal tip part of the endoscope when the insertion tube forms part of the endoscope and the bending section is in the retroflexion state, and
- marking the area in a colour configured to reduce over- or underexposure of the vision device of the distal tip part of the endoscope thereby providing a coloured area on exterior surface of the insertion tube.

By marking the area that is visible during retroflexion with an optimised colour, the vision device may be able to provide the operator with an improved image quality.

Additionally or alternatively, the method comprises a step of assembling the endoscope part with the remaining parts of the endoscope to obtain the endoscope.

Additionally or alternatively, the insertion tube comprises one or more additives configured for being activated in a laser marking process.

This may allow the provision different shadings and/or colourings to the coloured area, e.g. providing a gradual colouring.

Additionally or alternatively, the coloured area may be marked by a laser in a laser marking process.

Additionally or alternatively, the step of marking the area may comprise causing the laser to at least partially melt the exterior surface of the insertion tube within the area during the laser marking process. This may reduce the reflectivity of the area and thus reduce over- or underexposure.

It has been have found that over- or underexposure of the vision device is likely caused by a combination of the colour and the reflectivity of the exterior surface. By marking the coloured area using a laser marking process may also reduce the reflectivity of the surface, thus further allowing the vision device to optimise the exposure level to prevent loss of image detail. Only a slight laser treatment may be required to significantly reduce the reflectivity of the exterior surface.

Additionally or alternatively, the method may further comprise a step of marking two or more depth marks, optionally equidistantly, along the extent of the insertion tube.

Additionally or alternatively, the method may comprise a step of marking a quality inspection mark on the insertion tube indicating that the endoscope has passed quality inspection.

Additionally or alternatively, the coloured area, the depth marks, and optionally the quality inspection mark may be marked using the same marking process, preferably the same laser marking process, optionally either sequentially by using the same laser or simultaneously by using different lasers.

Such a method may improve the manufacturing efficiency since the coloured area, the depth marks, and optionally the quality inspection mark can be provided in the same process.

The features discussed in the present disclosure are particularly applicable to single use endoscopes, which are disposable and not intended to be cleaned and reused. The features discussed in the present disclosure may, however, also be applied to reusable endoscopes.

A person skilled in the art will appreciate that any one or more of the above aspects of this disclosure and embodiments thereof may be combined with any one or more of the other aspects of this disclosure and embodiments thereof.

### BRIEF DESCRIPTION OF THE DRAWINGS

Embodiments of this disclosure will be described in more detail in the following with regard to the accompanying figures. The figures show one way of implementing the present disclosure and are not to be construed as being limiting to other possible embodiments falling within the scope of the attached claim set.
Fig. 1 is a schematic perspective illustration of an endoscope according to this disclosure,
Fig. 2 is a schematic perspective illustration of a monitor connectable to the endoscope,
Fig. 3 is a schematic illustration of the endoscope in a retroflexion state, and
Fig. 4 is a schematic illustration of the distal end of the insertion tube in the retroflexion state.

### DETAILED DESCRIPTION

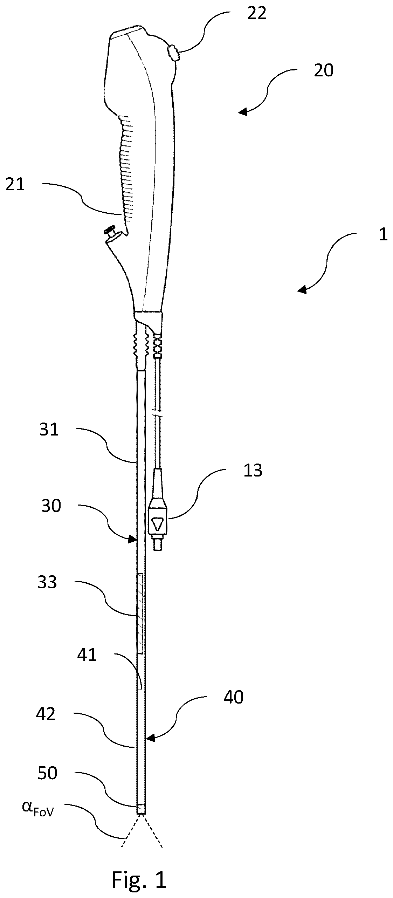

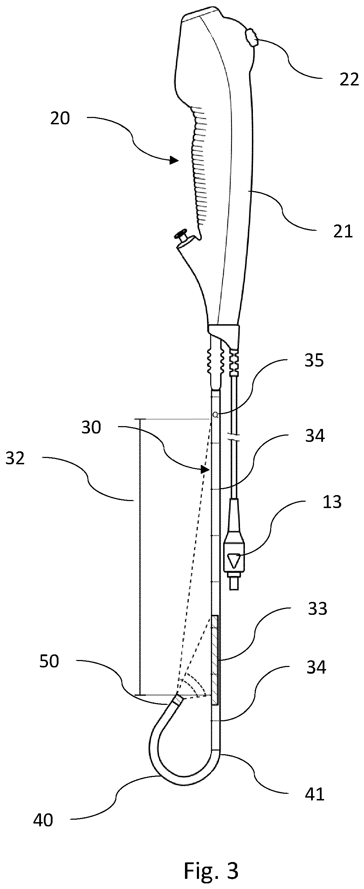

Fig. 1 illustrates an endoscope 1 which is disposable and not intended to be cleaned and reused. The endoscope 1 comprises a distal tip part 50, a handle 20, an insertion tube 30, and a bending section 40. The handle 20 includes a handle housing 21 for gripping and a control mechanism 22 in the form of a control lever. The insertion tube 30 is for insertion into a patient and extends between the handle 20 and a proximal end 41 of the bending section 40. Two steering wires (not shown) connect the control lever 22 with the bending section 40. A vision device (not shown), which in this case is in the form of a camera, and illumination means, such as LED's or light guides/fibre optics (not shown), are positioned in the distal tip part 50. The insertion tube 30 has an exterior tubular surface 31 facing the surroundings of the endoscope 1. In the present embodiment, the camera has a field of view angle α_{FoV} of about 60°. The control lever 22 is rotatable around a pivot axis to effect a bending movement of the bending section 40 in the plane of the Figs. 1 and 3-4 by tensioning either of the steering wires so as to bring the bending section 40 from the first state in which the bending section 40 is unbent and resting as shown in Fig. 1 to a retroflexion state as shown in Figs. 3-4. The camera is in signal communication with a circuit (not shown) of the handle 20 via data and power cables (not shown). The bending section 40 comprises segments (not shown), hinges (not shown) connecting the segments, and a thin outer sleeve 42 covering the segments and hinges and providing an additional layer of sealing for the connection between the distal tip part 50 and the bending section 40 and also provides a smooth outer surface for the bending section 40 in order to improve the comfort of a patient undergoing endoscopy.

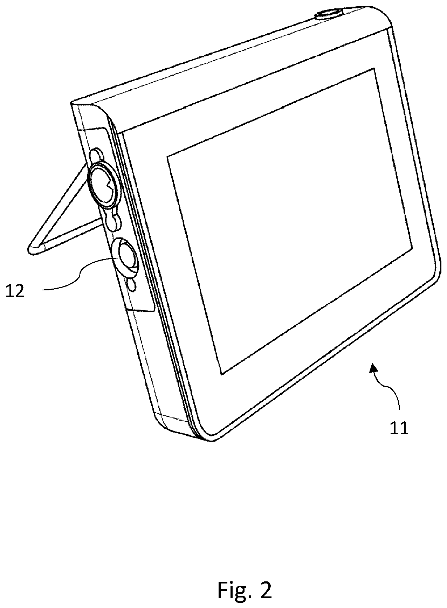

In Fig. 2, a monitor 11 is shown. The monitor 11 comprises a cable socket 12 to which a monitor cable 13 of the endoscope 1 shown in Fig. 1 can be connected to establish signal communication between the camera of the endoscope 1 and the monitor 11 via the circuit of the handle 20. The monitor 11 thus allows an operator to view images captured by the camera.

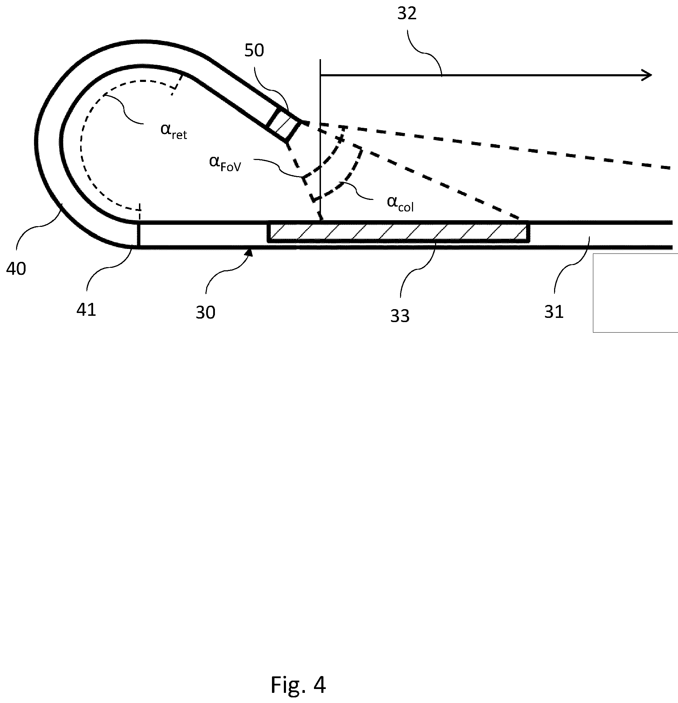

As mentioned, Fig. 3 shows the endoscope 1 in the retroflexion state and Fig. 4 shows a more detailed view of the distal end of the endoscope 1 in the retroflexion state. In the retroflexion state, the bending section 40 is bent to a retroflexion angle αᵣₑₜ of about 210° by manipulation of the control lever 22 to an extreme position, i.e. either fully up or fully down, to cause maximum tensioning of one of the steering wires so a first section 32 of the exterior surface 31 enters the field of view (FoV) of the camera. The first section 32 comprises a coloured area 33 covering approximately a distal third of the first section 32. In the retroflexion state, the field of view of the camera includes a view cone with an angle α_{col} of about 45° in which the visible portion of the exterior surface 31 is fully covered by the coloured area 33. This ensures that in the retroflexion state, the majority of the field of view of the camera and in particular the part of the exterior surface 31 closest to the camera is occupied by the coloured area 33 instead of the non-coloured remainder of the exterior surface 31. The coloured area 33 is configured to reduce over- or underexposure of the camera by being of a grey colour with L* between 15 to 75, e.g. 50, a* between negative 10 to positive 10, e.g. 0, and b* between negative 10 to positive 10, e.g. 0, as measured by the CIE L*a*b* colour code system. The exterior surface 31 further comprises a plurality of, e.g. eight, depth marks 34 spaced equidistantly along the extent of the insertion tube 30. Further, a quality inspection mark 35 may be provided, e.g. at a proximal end of the exterior surface 31, indicating that the endoscope 1 has passed quality inspection. The depth marks 34 and/or the quality inspection mark 35 are preferably of the same colour as the coloured area 33.

The coloured area 33, the depth marks 34, and the quality inspection mark 35 are provided by the following method.

Firstly, the insertion tube 30, which is configured for insertion into a patient and which includes the exterior surface 31 facing the surroundings is provided. Then an area of the exterior surface 31, which is visible within a field of view of the camera of the endoscope when the insertion tube 30 forms part of the endoscope 1 and the bending section 40 is in the retroflexion state, is located. A laser then marks the area in the grey colour, thereby providing the coloured area 33 on the insertion tube 30. The grey colour has an L* between 15 to 75, e.g. 50, a* between negative 10 to positive 10, e.g. 0, and b* between negative 10 to positive 10, e.g. 0, as measured by the CIE L*a*b* colour code system. The intensity of the grey colour can be adjusted by adjusting the power of the laser to achieve the desired greyness. Further, the insertion tube 30 can comprise additives to obtain different colours when exposed to light from the laser. The same laser is also used to mark the depth marks 34 and the quality inspection mark 35.

### LIST OF REFERENCES

The following is a list of reference numerals used throughout this disclosure. In case of any doubt, the reference numerals of the following list apply.
- 1: endoscope
- 11: monitor
- 12: cable socket
- 13: monitor cable
- 20: handle
- 21: handle housing
- 22: control lever
- 30: insertion tube
- 31: exterior surface
- 32: first section
- 33: coloured area
- 34: depth mark
- 35: quality inspection mark
- 40: bending section
- 41: proximal end
- 42: sleeve
- 50: distal tip part
- αᵣₑₜ: retroflex angle
- α_{FoV}: field of view angle
- α_{col}: field of view angle of the coloured area

## Claims

1. An endoscope (1) capable of performing a retroflexion manoeuvre, comprising:
- a handle (20) having a control mechanism (21),
- a distal tip part (50) having a vision device with a field of view, and an illumination device for providing illumination for the vision device,
- a bending section (40) having a first state, a retroflexion state, and a distal end connected to the distal tip part,
- an insertion tube (30) extending from the handle to a proximal end (41) of the bending section and including an exterior surface (31) facing the surroundings of the endoscope, and
- at least one steering wire connecting the control mechanism with the bending section so that manipulation of the control mechanism causes the endoscope to perform a retroflexion manoeuvre by bending the bending section from the first state to the retroflexion state,
wherein the exterior surface of the insertion tube includes a coloured area (33) of a colour, **characterised in that** the colour is configured to reduce over- or underexposure of the vision device when the bending section of the endoscope is in the retroflexion state, wherein the colour of the coloured area is a grey colour with L* between 15 to 85 as measured by a CIE L*a*b* colour code system.

2. An endoscope according to claim 1, wherein the coloured area is visible within the field of view of the vision device in the retroflexion state of the bending section.

3. An endoscope according to any one of the previous claims, wherein the field of view of the vision device includes a view cone with a view cone angle (α_{col}) of at least 10°, 20°, 30°, 40°, or 50° encompassing the coloured area within the field of view when the endoscope is in the retroflexion state.

4. An endoscope according to any one of the previous claims, wherein the colour of the coloured area is different from the colour of the majority of the insertion tube.

5. An endoscope according to any one of the previous claims, wherein the colour of the coloured area is a colour with L* between 30 to 70 as measured by a CIE L*a*b* colour code system.

6. An endoscope according to any one of the previous claims, wherein a first section of the exterior surface is visible within the field of view of the vision device in the retroflexion state of the bending section, and wherein the coloured area forms part of the first section and preferably covers at least 20%, 40%, 60%, 80%, 90%, or 100% of the area of the first section.

7. An endoscope according to claim 6, wherein the first section has a proximal portion and a distal portion, the coloured area covering the distal portion of the first section, the distal portion having a longitudinal extent of at most 90%, 80%, 70%, 60%, 50%, 40%, 30%, or 20% relative to a longitudinal extent of the exterior surface of the insertion tube.

8. An endoscope according to any one of the previous claims, wherein a bending angle (αᵣₑₜ) of the bending section between the first state and the retroflexion state is at least 180 degrees, such as 180-235 degrees, in particular 200-220 degrees, or preferably at least 210 degrees.

9. An endoscope according to any one of the previous claims, wherein the insertion tube comprises a plurality of depth marks (34), preferably spaced, optionally equidistantly, along the extent of the insertion tube, the depth marks being optionally of the same colour as the coloured area.

10. An endoscope vision system comprising an endoscope according to any one of the previous claims and a monitor, and wherein the endoscope is connectable to the monitor and the monitor includes a display unit configured for displaying images captured by the vision device of the endoscope.

11. A method for marking an area of an endoscope part for an endoscope capable of performing a retroflexion manoeuvre, the endoscope comprising:
- a handle (20) having a control mechanism (22);
- a distal tip part (50) having a vision device, such as a camera, with a field of view and an illumination device, such as one or more light emitting diodes (LEDs), for providing illumination for the vision device;
- a bending section (40) having a first state, a retroflexion state, and a distal end connected to the distal tip part;
- an insertion tube (30) extending from the handle to a proximal end of the bending section and including an exterior surface (31) facing the surroundings of the endoscope; and
- at least one steering wire connecting the control mechanism with the bending section so that manipulation of the control mechanism causes the endoscope to perform a retroflexion manoeuvre by bending the bending section from the first state to the retroflexion state;
wherein the method comprises the step of:
- providing the insertion tube configured for insertion into a patient and including the exterior surface facing the surroundings, and
**characterised in that** the method further comprises the steps of:
- locating an area of the exterior surface of the insertion tube being visible within a field of view of the vision device of the distal tip part of the endoscope when the insertion tube forms part of the endoscope and the bending section is in the retroflexion state, and
- marking the area in a colour configured to reduce over- or underexposure of the vision device of the distal tip part of the endoscope, thereby providing a coloured area (33) on the exterior surface of the insertion tube, wherein the colour is a grey colour with L* between 15 to 85 measured by a CIE L*a*b* colour code system.

12. A method according to claim 11, wherein the insertion tube comprises one or more additives configured for being activated in a laser marking process.

13. A method according to any one of claims 11-12, wherein the coloured area is marked by a laser in a laser marking process.

14. A method according to any one of claims 11-13, further comprising a step of:
- marking two or more depth marks (34), optionally equidistantly, along the extent of the insertion tube.

15. A method according to any one of claims 11-14 comprising a step of marking a quality inspection mark (35) on the insertion tube indicating that the endoscope has passed quality inspection.

16. A method according to claim 14 or 15 when dependent on claim 14, wherein the coloured area, the depth marks, and optionally the quality inspection mark are marked using the same marking process, preferably the same laser marking process, optionally either sequentially by using the same laser or simultaneously by using different lasers.

## Patentansprüche

1. Endoskop (1), das in der Lage ist, ein Retroflexionsmanöver durchzuführen, umfassend:
- einen Griff (20), der einen Steuermechanismus (21) aufweist,
- ein distales Spitzenteil (50), das eine Sichtvorrichtung mit einem Sichtfeld sowie eine Beleuchtungsvorrichtung zum Bereitstellen einer Beleuchtung für die Sichtvorrichtung aufweist,
- einen Biegeabschnitt (40) mit einem ersten Zustand, einem Retroflexionszustand und einem distalen Ende, das mit dem distalen Spitzenteil verbunden ist,
- ein Einführrohr (30), das sich vom Griff zu einem proximalen Ende (41) des Biegeabschnitts erstreckt und eine äußere Oberfläche (31) einschließt, die der Umgebung des Endoskops zugewandt ist, und
- mindestens einen Lenkdraht, der den Steuermechanismus mit dem Biegeabschnitt verbindet, sodass eine Betätigung des Steuermechanismus das Endoskop veranlasst, ein Retroflexionsmanöver durchzuführen, indem der Biegeabschnitt vom ersten Zustand in den Retroflexionszustand gebogen wird,
wobei die äußere Oberfläche des Einführrohrs einen farbigen Bereich (33) einer Farbe einschließt, **dadurch gekennzeichnet, dass** die Farbe dazu ausgebildet ist, eine Über- oder Unterbelichtung der Sichtvorrichtung zu verringern, wenn sich der Biegeabschnitt des Endoskops im Retroflexionszustand befindet, wobei die Farbe des farbigen Bereichs eine graue Farbe mit einem L* zwischen 15 und 85 ist, wie mit einem CIE-L*a*b*-Farbcodesystem gemessen.

2. Endoskop nach Anspruch 1, wobei der farbige Bereich im Sichtfeld der Sichtvorrichtung im Retroflexionszustand des Biegeabschnitts sichtbar ist.

3. Endoskop nach einem der vorstehenden Ansprüche, wobei das Sichtfeld der Sichtvorrichtung einen Sichtkegel mit einem Sichtkegelwinkel (α_{cal}) von mindestens 10°, 20°, 30°, 40° oder 50° einschließt, der den farbigen Bereich innerhalb des Sichtfelds umfasst, wenn sich das Endoskop im Retroflexionszustand befindet.

4. Endoskop nach einem der vorstehenden Ansprüche, wobei sich die Farbe des farbigen Bereichs von der Farbe des überwiegenden Teils des Einführrohrs unterscheidet.

5. Endoskop nach einem der vorstehenden Ansprüche, wobei die Farbe des farbigen Bereichs eine Farbe mit einem L* zwischen 30 und 70 ist, wie mit einem CIE-L*a*b*-Farbcodesystem gemessen.

6. Endoskop nach einem der vorstehenden Ansprüche, wobei ein erster Abschnitt der äußeren Oberfläche im Sichtfeld der Sichtvorrichtung im Retroflexionszustand des Biegeabschnitts sichtbar ist und wobei der farbige Bereich Teil des ersten Abschnitts ist und bevorzugt mindestens 20 %, 40 %, 60 %, 80 %, 90 % oder 100 % der Fläche des ersten Abschnitts abdeckt.

7. Endoskop nach Anspruch 6, wobei der erste Abschnitt einen proximalen Teil und einen distalen Teil aufweist, wobei der farbige Bereich den distalen Teil des ersten Abschnitts abdeckt, der eine Längserstreckung von höchstens 90 %, 80 %, 70 %, 60 %, 50 %, 40 %, 30 % oder 20 % relativ zu einer Längserstreckung der äußeren Oberfläche des Einführrohrs aufweist.

8. Endoskop nach einem der vorstehenden Ansprüche, wobei ein Biegewinkel (αᵣₑₜ) des Biegeabschnitts zwischen dem ersten Zustand und dem Retroflexionszustand mindestens 180 Grad beträgt, wie beispielsweise 180-235 Grad, insbesondere 200-220 Grad, oder bevorzugt mindestens 210 Grad.

9. Endoskop nach einem der vorstehenden Ansprüche, wobei das Einführrohr eine Vielzahl von Tiefenmarkierungen (34) umfasst, die bevorzugt entlang der Erstreckung des Einführrohrs beabstandet, wahlweise äquidistant, angeordnet sind, wobei die Tiefenmarkierungen wahlweise die gleiche Farbe wie der farbige Bereich haben.

10. Endoskop-Sichtsystem, umfassend ein Endoskop nach einem der vorstehenden Ansprüche, und einen Monitor, und wobei das Endoskop mit dem Monitor verbindbar ist und der Monitor eine Anzeigeeinheit einschließt, die dazu ausgebildet ist, von der Sichtvorrichtung des Endoskops erfasste Bilder anzuzeigen.

11. Verfahren zum Markieren eines Bereichs eines Endoskopteils für ein Endoskop, das in der Lage ist, ein Retroflexionsmanöver durchzuführen, wobei das Endoskop Folgendes umfasst:
- einen Griff (20), der einen Steuermechanismus (22) aufweist;
- ein distales Spitzenteil (50), das eine Sichtvorrichtung, wie beispielsweise eine Kamera, mit einem Sichtfeld, und eine Beleuchtungsvorrichtung, wie beispielsweise eine oder mehrere Leuchtdioden (LEDs), zum Bereitstellen einer Beleuchtung für die Sichtvorrichtung aufweist;
- einen Biegeabschnitt (40) mit einem ersten Zustand, einem Retroflexionszustand und einem distalen Ende, das mit dem distalen Spitzenteil verbunden ist;
- ein Einführrohr (30), das sich vom Griff zu einem proximalen Ende des Biegeabschnitts erstreckt und eine äußere Oberfläche (31) einschließt, die der Umgebung des Endoskops zugewandt ist; und
- mindestens einen Lenkdraht, der den Steuermechanismus mit dem Biegeabschnitt verbindet, sodass eine Betätigung des Steuermechanismus das Endoskop veranlasst, ein Retroflexionsmanöver durchzuführen, indem der Biegeabschnitt vom ersten Zustand in den Retroflexionszustand gebogen wird;
wobei das Verfahren den folgenden Schritt umfasst:
- Bereitstellen des Einführrohrs, das zum Einführen in einen Patienten ausgebildet ist und eine der Umgebung zugewandte äußere Oberfläche einschließt, und
**dadurch gekennzeichnet, dass** das Verfahren die folgenden Schritte umfasst:
- Auffinden eines Bereichs der äußeren Oberfläche des Einführrohrs, der innerhalb eines Sichtfelds der Sichtvorrichtung des distalen Spitzenteils des Endoskops sichtbar ist, wenn das Einführrohr Teil des Endoskops bildet und der Biegeabschnitt im Retroflexionszustand ist, und
- Markieren des Bereichs in einer Farbe, die dazu ausgebildet ist, eine Über- oder Unterbelichtung der Sichtvorrichtung des distalen Spitzenteils des Endoskops zu verringern, wodurch ein farbiger Bereich (33) auf der äußeren Oberfläche des Einführrohrs bereitgestellt wird, wobei die Farbe eine graue Farbe mit einem L* zwischen 15 und 85 ist, gemessen mit einem CIE-L*a*b*-Farbcodesystem.

12. Verfahren nach Anspruch 11, wobei das Einführrohr ein oder mehrere Additive umfasst, die dazu ausgebildet sind, in einem Laser-Markierungsverfahren aktiviert zu werden.

13. Verfahren nach einem der Ansprüche 11-12, wobei der farbige Bereich durch einen Laser in einem Laser-Markierungsverfahren markiert wird.

14. Verfahren nach einem der Ansprüche 11-13, weiter umfassend einen folgenden Schritt:
- Markieren von zwei oder mehr Tiefenmarkierungen (34), wahlweise äquidistant, entlang der Erstreckung des Einführrohrs.

15. Verfahren nach einem der Ansprüche 11-14, umfassend einen Schritt des Markierens einer Qualitätsprüfmarkierung (35) auf dem Einführrohr, die anzeigt, dass das Endoskop die Qualitätsprüfung bestanden hat.

16. Verfahren nach Anspruch 14 oder 15, sofern abhängig von Anspruch 14, wobei der farbige Bereich, die Tiefenmarkierungen und wahlweise die Qualitätsprüfmarkierung unter Verwendung desselben Markierungsverfahrens markiert werden, bevorzugt desselben Laser-Markierungsverfahrens, wahlweise entweder nacheinander unter Verwendung desselben Lasers oder gleichzeitig unter Verwendung unterschiedlicher Laser.

## Revendications

1. Endoscope (1) capable d'effectuer une manœuvre de rétroflexion, comprenant :
- une poignée (20) présentant un mécanisme de commande (21),
- une partie de pointe distale (50) présentant un dispositif de vision avec un champ de vision, et un dispositif d'éclairage pour fournir un éclairage pour le dispositif de vision,
- une section de courbure (40) présentant un premier état, un état de rétroflexion, et une extrémité distale connectée à la partie de pointe distale,
- un tube d'insertion (30) s'étendant de la poignée à une extrémité proximal (41) de la section de courbure et incluant une surface extérieure (31) faisant face aux alentours de l'endoscope, et
- au moins un câble de direction connectant le mécanisme de commande avec la section de courbure de sorte qu'une manipulation du mécanisme de commande amène l'endoscope à effectuer une manœuvre de rétroflexion en courbant la section de courbure du premier état à l'état de rétroflexion,
dans lequel la surface extérieure du tube d'insertion inclut une zone colorée (33) d'une couleur, **caractérisé en ce que** la couleur est configurée pour réduire la surexposition ou la sous-exposition du dispositif de vision lorsque la section de courbure de l'endoscope est dans l'état de rétroflexion, dans lequel la couleur de la zone colorée est une couleur grise avec L* entre 15 et 85 telle que mesurée par un système de code couleur CIE L*a*b*.

2. Endoscope selon la revendication 1, dans lequel la zone colorée est visible à l'intérieur du champ de vision du dispositif de vision dans l'état de rétroflexion de la section de courbure.

3. Endoscope selon l'une quelconque des revendications précédentes, dans lequel le champ de vision du dispositif de vision inclut un cône de vision avec un angle de cône de vision (α_{col}) d'au moins 10°, 20°, 30°, 40° ou 50°, englobant la zone colorée à l'intérieur du champ de vision lorsque l'endoscope est dans l'état de rétroflexion.

4. Endoscope selon l'une quelconque des revendications précédentes, dans lequel la couleur de la zone colorée est différente de la couleur de la majorité du tube d'insertion.

5. Endoscope selon l'une quelconque des revendications précédentes, dans lequel la couleur de la zone colorée est une couleur avec L* entre 30 et 70 telle que mesurée par un système de code couleur CIE L*a*b*.

6. Endoscope selon l'une quelconque des revendications précédentes, dans lequel une première section de la surface extérieure est visible à l'intérieur du champ de vision du dispositif de vision dans l'état de rétroflexion de la section de courbure, et dans lequel la zone colorée fait partie de la première section et couvre de préférence au moins 20 %, 40 %, 60 %, 80 %, 90 % ou 100 % de la zone de la première section.

7. Endoscope selon la revendication 6, dans lequel la première section présente une partie proximale et une partie distale, la zone colorée couvrant la partie distale de la première section, la partie distale présentant une étendue longitudinale d'au plus 90 %, 80 %, 70 %, 60 %, 50 %, 40 %, 30 % ou 20 % par rapport à une étendue longitudinale de la surface extérieure du tube d'insertion.

8. Endoscope selon l'une quelconque des revendications précédentes, dans lequel un angle de courbure (αᵣₑₜ) de la section de courbure entre le premier état et l'état de rétroflexion est au moins 180 degrés, tel que 180-235 degrés, en particulier 200-220 degrés, ou de préférence au moins 210 degrés.

9. Endoscope selon l'une quelconque des revendications précédentes, dans lequel le tube d'insertion comprend une pluralité de repères de profondeur (34), de préférence espacés, éventuellement de manière équidistante, le long de l'étendue du tube d'insertion, les repères de profondeur étant éventuellement de la même couleur que la zone colorée.

10. Système de vision d'endoscope comprenant un endoscope selon l'une quelconque des revendications précédentes et un moniteur, et dans lequel l'endoscope peut être connecté au moniteur et le moniteur inclut une unité d'affichage configurée pour afficher des images capturées par le dispositif de vision de l'endoscope.

11. Procédé de marquage d'une zone d'une partie d'endoscope pour un endoscope capable d'effectuer une manœuvre de rétroflexion, l'endoscope comprenant :
- une poignée (20) présentant un mécanisme de commande (22) ;
- une partie de pointe distale (50) présentant un dispositif de vision, tel qu'une caméra, avec un champ de vision et un dispositif d'éclairage, tel qu'une ou plusieurs diodes électroluminescentes (LED), pour fournir un éclairage pour le dispositif de vision ;
- une section de courbure (40) présentant un premier état, un état de rétroflexion, et une extrémité distale connectée à la partie de pointe distale ;
- un tube d'insertion (30) s'étendant de la poignée à une extrémité proximale de la section de courbure et incluant une surface extérieure (31) faisant face aux alentours de l'endoscope ; et
- au moins un câble de direction connectant le mécanisme de commande avec la section de courbure de sorte qu'une manipulation du mécanisme de commande amène l'endoscope à effectuer une manoeuvre de rétroflexion en courbant la section de courbure du premier état à l'état de rétroflexion ;
dans lequel le procédé comprend l'étape consistant à :
- fournir le tube d'insertion configuré pour une insertion dans un patient et incluant la surface extérieure faisant face aux alentours, et
**caractérisé en ce que** le procédé comprend en outre les étapes consistant à :
- localiser une zone de la surface extérieure du tube d'insertion qui est visible à l'intérieur d'un champ de vision du dispositif de vision de la partie de pointe distale de l'endoscope lorsque le tube d'insertion fait partie de l'endoscope et que la section de courbure est dans l'état de rétroflexion, et
- marquer la zone dans une couleur configurée pour réduire la surexposition ou la sous-exposition du dispositif de vision de la partie de pointe distale de l'endoscope, fournissant ainsi une zone colorée (33) sur la surface extérieure du tube d'insertion, dans lequel la couleur est une couleur grise avec L* entre 15 et 85 mesurée par un système de code couleur CIE L*a*b*.

12. Procédé selon la revendication 11, dans lequel le tube d'insertion comprend un ou plusieurs additifs configurés pour être activés dans un processus de marquage laser.

13. Procédé selon l'une quelconque des revendications 11 à 12, dans lequel la zone colorée est marquée par un laser dans un processus de marquage laser.

14. Procédé selon l'une quelconque des revendications 11 à 13, comprenant en outre une étape consistant à :
- marquer deux ou plusieurs repères de profondeur (34), éventuellement de manière équidistante, le long de l'étendue du tube d'insertion.

15. Procédé selon l'une quelconque des revendications 11 à 14, comprenant une étape de marquage d'un repère d'inspection de qualité (35) sur le tube d'insertion indiquant que l'endoscope a passé une inspection de qualité.

16. Procédé selon la revendication 14 ou 15 lorsqu'elle dépend de la revendication 14, dans lequel la zone colorée, les repères de profondeur, et éventuellement la marque d'inspection de qualité sont marqués en utilisant le même processus de marquage, de préférence le même processus de marquage laser, éventuellement soit séquentiellement en utilisant le même laser soit simultanément en utilisant des lasers différents.
